(19) Europäisches Patentamt
European Patent Office
Office européen des brevets

(11) **EP 2 424 523 B1**

(12) **EUROPEAN PATENT SPECIFICATION**

(45) Date of publication and mention of the grant of the patent:
**27.06.2012 Bulletin 2012/26**

(21) Application number: **10715337.1**

(22) Date of filing: **23.04.2010**

(51) Int Cl.:
**A61K 31/335** (2006.01)   **A61K 9/12** (2006.01)

(86) International application number:
**PCT/GB2010/050672**

(87) International publication number:
**WO 2010/122356 (28.10.2010 Gazette 2010/43)**

(54) **SUBLINGUAL SPRAY FORMULATION COMPRISING DIHYDROARTEMESININ**

SUBLINGUALE SPRAY-FORMULIERUNG MIT DIHYDROARTEMESININ

FORMULATION DE PULVÉRISATION SUBLINGUALE COMPRENANT DE LA DIHYDROARTÉMÉSININE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **23.04.2009  GB 0906971**
**23.04.2009  PCT/GB2009/050415**

(43) Date of publication of application:
**07.03.2012  Bulletin 2012/10**

(73) Proprietor: **Londonpharma Ltd.**
**Colney Lane, Norwich NR4 7UH (GB)**

(72) Inventor: **ROSS, Calvin John**
**Deceased (GB)**

(74) Representative: **ip21 Ltd**
**Central Formalities Department**
**Lakeside 300**
**Old Chapel Way**
**Broadland Business Park**
**Norwich**
**Norfolk NR7 0WG (GB)**

(56) References cited:
**US-A1- 2006 233 895**

• **TOUITOU E ET AL:** "Treatment of malaria in a mouse model by intranasal drug administration" INTERNATIONAL JOURNAL OF PARASITOLOGY, PERGAMON PRESS, GB LNKD- DOI:10.1016/J.IJPARA.2006.07.006, vol. 36, no. 14, 1 December 2006 (2006-12-01), pages 1493-1498, XP025241521 ISSN: 0020-7519 [retrieved on 2006-12-01]
• **JANSE C J ET AL:** "Comparison of in vivo and in vitro antimalarial activity of artemisinin, dihydroartemisinin and sodium artesunate in the Plasmodium berghei-rodent model" INTERNATIONAL JOURNAL OF PARASITOLOGY, PERGAMON PRESS, GB LNKD- DOI:10.1016/0020-7519(94)90150-3, vol. 24, no. 4, 1 July 1994 (1994-07-01), pages 589-594, XP023651854 ISSN: 0020-7519 [retrieved on 1994-07-01]
• **SHALABY H A ET AL:** "In vitro effect of artemether and triclabendazole on adult Fasciola gigantica" VETERINARY PARASITOLOGY, ELSEVIER SCIENCE, AMSTERDAM, NL LNKD- DOI: 10.1016/J.VETPAR.2008.10.027, vol. 160, no. 1-2, 9 March 2009 (2009-03-09), pages 76-82, XP025938029 ISSN: 0304-4017 [retrieved on 2008-10-17]
• **KEISER ET AL:** "Food-borne trematodiasis: current chemotherapy and advances with artemisinins and synthetic trioxolanes" TRENDS IN PARASITOLOGY, ELSEVIER CURRENT TRENDS, GB, vol. 23, no. 11, 22 October 2007 (2007-10-22), pages 555-562, XP022326691 ISSN: 1471-4922

• NAKASE ET AL: "Anticancer properties of artemisinin derivatives and their targeted delivery by transferrin conjugation" INTERNATIONAL JOURNAL OF PHARMACEUTICS, ELSEVIER BV, NL LNKD-DOI:10.1016/J.IJPHARM.2007.09.003, vol. 354, no. 1-2, 20 March 2008 (2008-03-20), pages 28-33, XP022550487 ISSN: 0378-5173

• NAM WOONG ET AL: "Effects of artemisinin and its derivatives on growth inhibition and apoptosis of oral cancer cells" HEAD & NECK, vol. 29, no. 4, April 2007 (2007-04), pages 335-340, XP002588131 ISSN: 1043-3074

**Description**

Field of the Invention

The invention relates to pharmaceutical compositions, delivery methods, delivery devices and methods for the treatment of cancer. The invention also relates to pharmaceutical compositions, delivery methods, delivery devices and methods for the treatment of fluke infestations and Lyme disease (Borreliosis).

Background and Prior Art Known to the Applicant

Artemesinins, which may be isolated from the plant *Artemesia annua* are known for the treatment of malaria, and have also been shown to be effective for the treatment of a wide range of cancers, i.e. neoplasms, and especially malignant neoplasms. Amongst reported successes are the following:

Sing and Panwar (Integrative Cancer Therapies, 5(4): 2006, 391-394) report the treatment of pituitary adenoma with artemether.

Singh and Verma (Archive of Oncology, 10(4): 2002, 279-280) report the treatment of laryngeal squamous cell carcinoma with artesunate.

Singh and Lai (Life Sciences, 70(2001) 49-56) report the selective toxicity of dihydroartemesinin and holotransferrin toward human breast cancer cells.

Rowen (Townsend Letter for Doctors and Patients, December 2002) provides a summary of the use of artemisinins for the treatment of various cancers, including breast cancer, non-Hodgkin's Lymphoma, non-small cell lung carcinoma, and multiple skin cancers.

Efferth et al ("Anti-malaria drug is also active against cancer", Int. J. Oncology, 18; 767-773, 2001) report activity of artemesinins against 55 cancer lines.

It is believed that the artemesinins have this broad effect on a large range of cancer cells because of their ability to react with ferrous iron to form free radicals: and most cancer cells have high rates of iron intake.
In addition, artemesinins have been shown to be effective in the treatment of liver flukes, and in particular schistosomiasis. Keiser and Morson (Exp. Parasitol., 118(2), 2008: 228-37) report the activity of artesunate and artemether against the liver fluke *Fasciola hepatica.*
Keiser et al (J. Antimicrobial Chemotherapy, 2006, 57, 1139-1145) also report that artesunate and artemether are effective fasciolicides.
Utzinger et al (Curr Opin Investig Drugs, 2007 Feb 8(2), 105-16) report the use of artemesinins for treatment of individuals infested with *Plasmodium spp.* and *Schistosoma haematobium* with promising activity or artemesinins against intestinal and liver flukes, as well as against cancer cells.
Recent observations have also found that artemesinins are active against bacteria of the genus *Borrelia,* the causative agent of Lyme disease. *Borrelia burgdorferi* is the predominant cause of Lyme disease in the United States, *Borrelia afzelii* and *Borrelia garinii* being more common agents in most European cases.
Accordingly, amongst the active pharmaceutical of use in the treatment of these conditions are a number of compounds derived from artemesenin, a sesquiterpene lactone endoperoxide originally isolated from *Artemesia annua* (Woodrow et al. Postgrad. Med.J. 2005; 81:71-78). These compounds include the semi-synthetic derivatives artenimol, artesunate, artemether and arteether (artemotil). The International Pharmacopoeia (*Ph. Int.,* World Health Organisation) lists a number of these for the treatment of malaria (against which they are also active), viz: Artemether in the form of capsules, tablets or an injectable formulation; Artemesenin in the form of capsules or tablets; arteether in an injectable formulation; and both artenimol and artesunate in the form of tablets. Once taken into the body, the artemesinins are converted to dihydroartemesinin and so these active compounds include all those that supply dihydroartemesinin *in vivo.*
One particular problem with the administration of artemesinins is their low bioavailability and the presence of a first pass effect when taken by the oral route, as will be discussed below. Furthermore, for long-term cancer treatment, it is particularly preferred that patients are able to either self-administer medication, or that medication can be administered by a non-qualified helper, and particularly in the home environment. This allows patients to remain at home, and reduces pressure on the healthcare system. Furthermore, cancer patients are often immune-compromised, and it is therefore particularly beneficial to keep them out of e.g. a hospital environment where the chances of contracting infections are higher. For these reasons at least, oral doses of artemesinins are not effective, especially for long-term treatment as might be required for cancer therapy, for treatment of fluke infestations or treatment of Lyme disease; injectable treatments are prone to risk of infection, need medically-qualified personnel and are not stable during storage; suppository administration is also not acceptable in many cultures, and might not be repeatably absorbed where patients are experiencing diarrhoea.

It can be seen that all of these formulations face the difficulties of administration described above. It is therefore amongst the objects of the present invention to address these and other issues.

Summary of the Invention

Accordingly, in a first aspect, the invention provides a pharmaceutical composition for use in the treatment of a neoplastic disease, said composition comprising: artemesinin, artemether, arteether, artenimol or artesunate; and a pharmaceutically-acceptable excipient selected the group consisting of: medium chain length triglycerides; short chain triglycerides; omega-3-marine triglycerides; and fish oil, rich in omega-3-acids, said composition formulated for transmucosal sublingual, buccal or nasal dosage.

In a second aspect, the invention provides a pharmaceutical composition for use in the treatment of fluke infestation, said composition comprising: artemesinin, artemether, arteether, artenimol or artesunate; and a pharmaceutically-acceptable excipient selected the group consisting of: medium chain length triglycerides; short chain triglycerides; omega-3-marine triglycerides; and fish oil, rich in omega-3-acids, said composition formulated for transmucosal sublingual, buccal or nasal dosage.

In a third aspect, the invention provides a pharmaceutical composition for use in the treatment of Lyme disease (borreliosis), said composition comprising: artemesinin, artemether, arteether, artenimol or artesunate; and a pharmaceutically-acceptable excipient selected the group consisting of: medium chain length triglycerides; short chain triglycerides; omega-3-marine triglycerides; and fish oil, rich in omega-3-acids, said composition formulated for transmucosal sublingual, buccal or nasal dosage.

The inventors have found that the transmucosal sub-lingual, transmucosal buccal and transmucosal nasal routes for administration of artemether or arteether are effective for delivery of the pharmaceutical into the systemic circulation e.g. for the treatment of cancer and fluke infestation. Furthermore, for the first time, it provides an administration route that is acceptable to patients requiring treatment, and that may be administered by non-medically qualified personnel. It has particular advantage, therefore, in treating these conditions. The composition can be delivered e.g. sublingually as a liquid bolus, or, more preferably, as a spray.

Medium chain length triglycerides are defined in the European Pharmacopoeia Monograph 0868, as:

A mixture of triglycerides of saturated fatty acids, mainly of caprylic acid (octanoic acid, $C_8H_{16}O_2$) and of capric acid (decanoic acid, $C_{10}H_{20}O_2$). Medium-chain triglycerides are obtained from the oil extracted from the hard, dried fraction of the endosperm of *Cocos nucifera* L. or from the dried endosperm of *Elaeis guineensis* Jacq. When Medium-chain Triglycerides are prepared from the endosperm of *Cocos nucifera* L., the title Fractionated Coconut Oil may be used. Medium chain length triglycerides have a minimum 95.0 per cent of saturated fatty acids with 8 and 10 carbon atoms. Further chemical and physical properties are described in the European Pharmacopoeia Monograph 0868, and equivalent documents.

Short chain triglycerides are triglycerides having chain lengths of less than 6 carbon atoms.

Omega-3-marine triglycerides are defined in the European Pharmacopoeia Monograph 0868 as mixture of mono-, di- and triesters of omega-3 acids with glycerol containing mainly triesters and obtained either by esterification of concentrated and purified omega-3 acids with glycerol or by transesterification of the omega-3 acid ethyl esters with glycerol. The origin of the omega-3 acids is the body oil from fatty fish species coming from families like *Engraulidae, Carangidae, Clupeidae, Osmeridae, Salmonidae* and *Scombridae.* The omega-3 acids are identified as the following acids: alpha-linolenic acid (C18:3 n-3), moroctic acid (C18:4 n-3), eicosatetraenoic acid (C20:4 n-3), timnodonic (eicosapentaenoic) acid (C20:5 n-3; EPA), heneicosapentaenoic acid (C21:5 n-3), clupanodonic acid (C22:5 n-3) and cervonic (docosahexaenoic) acid (C22:6 n-3; DHA). The sum of the contents of the omega-3 acids EPA and DHA, expressed as triglycerides is a minimum of 45.0 per cent, and the total omega-3 acids, expressed as triglycerides is a minimum of 60.0 per cent. Tocopherol may be added as an antioxidant.

Fish oil, rich in omega-3-acids is also defined in the European Pharmacopeia as purified, winterised and deodorised fatty oil obtained from fish of the families *Engraulidae, Carangidae, Clupeidae, Osmeridae, Scombridae* and *Ammodytidae.* The omega-3 acids are defined as the following acids: *alpha*-linolenic acid (C18:3 n-3), moroctic acid (C 18:4 n-3), eicosatetraenoic acid (C20:4 n-3), timnodonic (eicosapentaenoic) acid (C20:5 n-3; EPA), heneicosapentaenoic acid (C21:5 n-3), clupanodonic acid (C22:5 n-3) and cervonic (docosahexaenoic) acid (C22:6 n-3; DHA).

The content of the Fish oil, rich in omega-3-acids is as follows:

EPA, expressed as triglycerides: minimum 13.0 per cent,
DHA, expressed as triglycerides: minimum 9.0 per cent,

Total omega-3-acids, expressed as triglycerides: minimum 28.0 per cent.

Authorized antioxidants in concentrations not exceeding the levels specified by the competent authorities may be added.

Whilst these definitions serve to define particularly preferred compositions of the recited excipients, the skilled addressee will appreciate that the composition of appropriate alternative excipients may also deviate from these exact compositional limits. Excipients of choice should exhibit analogous chemical properties such as the ability to solubilise artemether or arteether or other compounds providing dihydroartemesinin at the required concentration, not to degrade the pharmaceutically active ingredients, and to be non-toxic. The excipients should also have analogous physical properties such as at least being liquid at body temperature, and preferably having a suitable viscosity to allow the excipient to be used in preferred spray formulations described below. The viscosity for these applications should be low enough to be capable of atomizing, as described below, when used in a pump spray.

As an example, compositions might consist essentially of artemether or arteether and a pharmaceutically acceptable excipient consisting essentially of a triglyceride, liquid at 37°C, and medium chain triglycerides (as defined herein).

Particularly preferred compositions consist essentially of: artemether or arteether; and one or more pharmaceutically-acceptable excipients selected the group consisting of: medium chain length triglycerides; short chain triglycerides; and omega-3-marine triglycerides, said composition formulated for transmucosal sublingual, buccal or nasal dosage. The exclusion of significant amounts of other materials (e.g. higher molecular weight lipids) renders a composition that is ideally suited to transmucosal nasal, buccal, and especially sublingual delivery.

More preferred compositions comprise: artemether and a pharmaceutically-acceptable excipient selected the group consisting of: medium chain length triglycerides; short chain triglycerides; and omega-3-marine triglycerides, said composition formulated for transmucosal sublingual, buccal or nasal dosage, and especially a composition consisting essentially of: artemether and a pharmaceutically-acceptable excipient selected the group consisting of: medium chain length triglycerides; short chain triglycerides; and omega-3-marine triglycerides, said composition formulated for transmucosal sublingual, buccal or nasal dosage.

In any of these compositions, it is especially preferred that the composition is substantially free of water, as the inventors have found, contrary to accepted belief, that water can significantly reduce the shelf-life of the compositions, especially when stored at ambient temperatures. Preferred compositions would have less than 1%(w/w) water, and more preferably less than 0.5%(w/w) water, and most preferably less than 0.1 %(w/w) water.

Also in any of these compositions, it is especially preferred that the composition is substantially free of ethanol. Again, the inventors have found that ethanol leads to degradation of the pharmaceutically active components. Preferred compositions in particular have less than 1%(w/w) ethanol, and more preferably less than 0.5%(w/w) ethanol and most preferably less than 0.1%(w/w) ethanol.

Also in any of these compositions, it is preferred that artemether or arteether is present at a concentration of between 2 and 250 milligrams per gram of excipient. This concentration provides an appropriate level for the expected volumes used for the described transmucosal delivery. More preferably, the composition comprises: artemether or arteether, dissolved in the excipient at a concentration of between 2 and 200 milligrams per gram of excipient. Other preferred concentrations are between 2 and 100 milligrams per gram; between 2 and 50 milligrams per gram. The lower concentrations provide compositions particularly suitable for paediatric use, and are also more likely to ensure that the pharmaceutically active components remain in solution over a wide temperature range, rather than having some portion as e.g. a suspension. This is particularly important to ensure that delivery of the drug is by the recited transmucosal route. If significant amounts of the active components are not in solution, then there is an increased likelihood that some will be swallowed, thereby reducing the beneficial effects of such transmucosal delivery described below.

In especially preferred compositions, the said excipient comprises a medium chain triglyceride, said triglyceride comprising a minimum of 95 per cent of saturated fatty acids with between 6 and 12 carbon atoms. More preferably, said excipient comprises a medium chain triglyceride, said triglyceride comprising a minimum of 95 per cent of saturated fatty acids with between 8 and 10 carbon atoms.

Also in any such composition, it is also particularly preferred that the composition further comprises an essential oil such as menthol, vanillin or orange oil, lemon oil, clove oil, peppermint oil, spearmint oil. Particular technical advantages of such an essential oil, especially menthol, which acts as a solubilising agent, are described further below. In addition to any solubilising effect such essential oils also act as flavourings, having a number of benefits: the flavours mask unpleasant tastes of the medicament thereby leading to increased patient compliance. This is particularly important for such essentially liquid-based formulations which cannot by their nature be encapsulated or "sugar-coated". The flavours also give a feedback to the user or administrator of the medication that the medication has been successfully delivered (the patient can taste it), and furthermore that it has been delivered to the correct place.

In a further aspect, the invention provides compositions as described herein, for any of said uses, that are contained within a medicament delivery device, said device adapted to deliver individual or successive doses of said composition, each individual or successive dose having a volume of less than 1000 microlitres. The use of small dose volumes reduces the likelihood that the composition will be swallowed, or spat out, by the patient. The likelihood is reduced further by use of smaller volumes (especially in the paediatric context or for nasal delivery) and so in further preferred embodiments, each successive dose has a volume of less than 600 microlitres; less than 400 microlitres; less than 200 microlitres; or even less than 100 microlitres. Smaller volumes are especially preferred for paediatric use, or nasal delivery.

In an additional aspect, the invention provides compositions as described herein, for any of said uses, that are contained within a medicament delivery device, said device adapted to deliver individual or successive doses of said composition, each individual or successive dose containing no more than 100mg, and preferably no more than 80mg of a compound capable of providing dihydroartemesinin, such as artemether or arteether. Such devices are preferably adapted to assist sublingual delivery, especially by non-medically trained personnel. Limiting the amount of active pharmaceutical delivered with each dose is especially important in the context of treatment by less skilled personnel (e.g. self-administration by a patient in a domestic setting, which is likely for long-term anti cancer therapy) to ensure that over-dosing is avoided. Preferably, said device and composition adapted to deliver individual or successive doses of said composition, each individual or successive dose containing no more than 10mg of a compound capable of providing dihydroartemesinin, such as artemether or arteether. This provides an appropriate device for paediatric use.

Preferably, the delivery devices according to these aspects comprise a spray, and especially a pump spray. The use of a pump spray increases the area of mucosa to which the composition is applied, thereby increasing absorption and minimising the likelihood that the medicament is swallowed. More preferably, said device is adapted to produce a spray of composition having a mean droplet diameter greater than 20 microns, or even greater than 50 microns, or preferably greater than 75 microns. In this way, inadvertent delivery of the medicament to the lungs is avoided, or reduced.

In another aspect, the invention also provides compositions as described herein, for any of said uses, that are contained within a container comprised within a device for providing pharmaceutical with valve means arranged to transfer doses of said pharmaceutical composition to the exterior of the container. Such a device may be attached to e.g. a separate transmucosal buccal, nasal or sublingual delivery device, such as a spray.

In a final aspect, the invention provides compositions as described herein, for any of said uses, that are contained within a kit that comprises instructions to administer said composition to a patient in need thereof by the transmucosal sublingual, buccal or nasal route. Preferably, said kit has instructions to administer said composition to a patient in need thereof by the sublingual route.

It is particularly preferred that the composition also includes a transferrin, such as holotransferrin, as this enhances the action of dihydroartemesinin.

Description and Preferred Embodiments of the Invention

One of the most important aspects of providing a clinically useful treatment for diseases, infections or infestations responsive to dihydroartemesinin (produced *in vivo* by metabolisms of an artemesinin such as artemether, arteether and artesunate) is to provide a formulation and an administration route for the active ingredient that can withstand the challenges of those communities where the disease is an especially acute problem. For example, any formulation needs to be stable for long periods of time, and at the relatively high temperatures encountered in countries where e.g. schistosomiasis is endemic. The medicament will often need to be administered (without delay) to individuals who are weak, perhaps malnourished, and possibly suffering from vomiting and diarrhoea. In many cases, the medicament may also need to be administered by non-medically-trained personnel. It is also important for any active ingredient to have good (and consistent) bioavailability, to ensure that the drug reaches the site of action without adverse side effects.

In order to address these problems, the inventors have found that the transmucosal sublingual, buccal or nasal route of administration of artemether provides a greater likelihood of higher and more reproducible levels of bioavailability than that demonstrated by the oral (i.e. swallowed) or intramuscular route. Navaratnam et al (Clin Pharmacokinet, 2000, Oct; 39(4): 255-270) report the bioavailability of artemether in animals by oral administration to be as low as 19-35%, and only 54% when administered by intramuscular injection. In humans, the bioavailability of artemether was low in both the intramuscular (25%) and intrarectal (35%) route, with considerable variability in absorption. The authors report that *"Preliminary studies in children with cerebral malaria indicated that the bioavailability of intramuscular artemether is highly variable and could potentially effect treatment outcome in the most severely ill patients"*.

The use of the transmucosal sublingual, buccal or nasal route of administration avoids the first-pass effect that occurs with oral and rectal administration. Whilst adults might be able to tolerate the large oral doses of artemether required to overcome the low bioavailability of the drug for short periods of time, this is not the case in children, and so the compositions disclosed herein are particularly suitable for the treatment of diseases such as cancer that might require protracted periods of medication, or that might be for paediatric use.

Preliminary results of initial, confidential, dose ranging studies are presented below, indicating surprisingly increased bioavailability of the drug when administered by sublingual spray in comparison to oral administration by tablet.

The inventors have also found that, contrary to accepted belief, artemether is not stable when in contact with water, ethanol, or propellants that might be used for aerosol formulations.

Tables 1 and 2 show impurities present in Artemether API, and artemether in three solvent systems: 20% ethanol + 80% propellant; 50% ethanol + 50% propellant; 100% ethanol; and a medium chain triglyceride, in this case, the triglyceride sold under the registered trade mark Miglyol® 810. Miglyol® is a medium chain triglyceride containing saturated C8 and C10 fatty acids, typically between 65-80% of caprylic acid (C8:0) and 20-35% of capric acid (C10:0).

The propellant used in these test was 1,1,1,2 tetrafluoroethane, sold under the registered trade mark Zephex® 134a. Similar results were obtained for the propellants butane, Zephex® 227 (1,1,1,2,3,3,3 heptafluoropropane) and for a mixture of butane and propane.

Table 1 shows the impurities (as a percentage of the peak area of an HPLC chromatogram of artemether) after storage of the compositions at 30°C for eight weeks. Table 2 shows the corresponding impurities after storage for eight weeks at 40°C.

*Table 1- Storage at 30°C*

| Relative Retention Time: | 0.35 | 0.68 | 0.73 | 0.87 | 0.91 | 1.17 |
| --- | --- | --- | --- | --- | --- | --- |
| | % of artemether | | | | | |
| Artemether API | 0.4 | | 0.1 | 0.2 | | |
| 20% EtOH 80% propellant | 1.6 | 0.3 | 0.7 | 0.2 | 1.3 | 0.2 |
| 50% EtOH 50% propellant | 1.0 | 0.2 | 0.5 | 0.2 | 1.5 | 0.2 |
| 100% EtOH | 0.3 | 0.2 | 0.5 | 0.2 | | |
| Miglyol 810® | 0.4 | | 0.1 | 0.2 | | |

*Table 2 - Storage at 40°C*

| Relative Retention Time: | 0.35 | 0.68 | 0.73 | 0.87 | 0.91 | 1.17 |
| --- | --- | --- | --- | --- | --- | --- |
| | % of artemether | | | | | |
| Artemether API | 0.4 | | 0.1 | 0.2 | | |
| 20% EtOH 80% propellant | 4.9 | 1.9 | 2.9 | 0.2 | 5.3 | 1.4 |
| 50% EtOH 50% propellant | 2.2 | 1.4 | 2.5 | 0.2 | 4.8 | 1.0 |
| 100% EtOH | 2.2 | 0.7 | 1.6 | 0.2 | 1.0 | 0.7 |
| Miglyol 810® | 0.6 | | 0.1 | 0.2 | | |

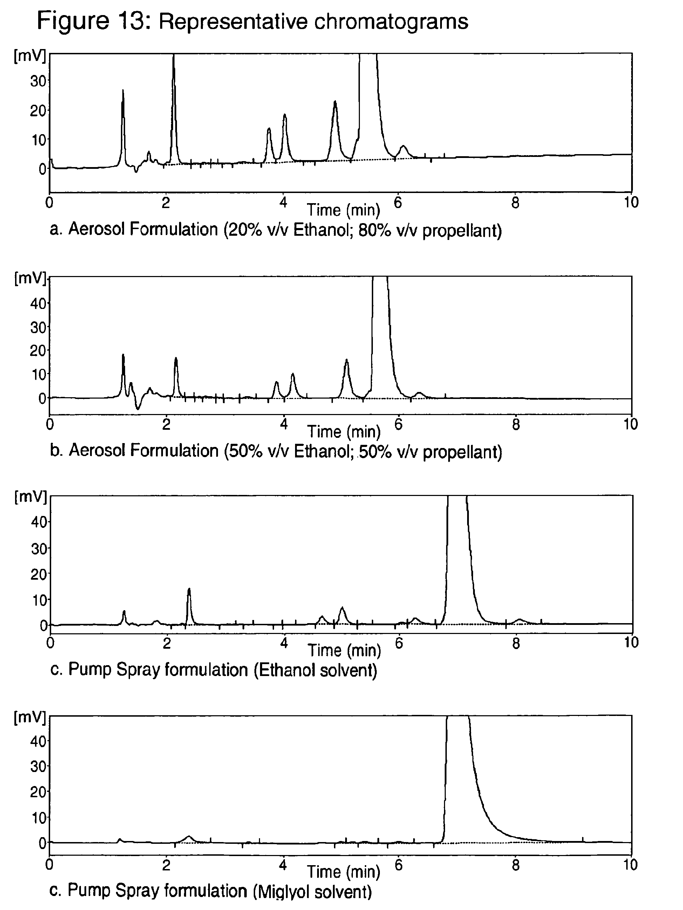

Representative chromatograms are shown in Figure 13. It can be seen that the levels of impurities in the Miglyol® 810 formulation are not significantly higher than those observed in the initial Artemether API. In all other cases, the impurities are at levels that exceed those permitted under the ICH Harmonised Tripartite Guidelines for Impurities in New Drug Products without specific identification or further toxicological examination.

A solution in a medium chain triglyceride, especially a saturated triglyceride such as Miglyol® 810 therefore constitutes a stable formulation for the active ingredient. Being a saturated triglyceride, it is believed that this confers stability to the artemether. Given its chemical structure, it is likely that the main route of degradation of artemether is via reduction mechanisms, which might explain the protection afforded by such saturated fatty acid-containing triglycerides.

When used in a spray delivery system, e.g. in a manually-actuated pump spray, the triglyceride also acts as a pump and valve lubricant, thereby removing the need to add additional lubricants to the formulation. The use of such medium chain triglycerides also produces a formulation of appropriate viscosity and surface tension for use in a pump spray delivery system.

Further advantages also flow from the use of medium chain triglyceride: being hydrophobic, the triglyceride adheres to the mucosa of the mouth, and so allows time for the artemether to be absorbed transmucosally. The hydrophobic nature of the composition resists being washed out of the mouth by the action of saliva, which would otherwise cause the active ingredient to be swallowed.

In especially preferred embodiments of the invention, the artemether-triglyceride solution is supplemented with menthol, or alternatively with orange oil or vanilla. The inventors have found that this has a number of benefits:

(1) Its function as a taste-masking agent is particularly important in the context of administration of drugs to children or to patients who need to take the medication over prolonged periods of time; any bad taste of the drug experienced by the patient makes patient compliance less likely.

(2) The essential oil also acts as a penetration enhancer to improve the uptake of the pharmaceutical ingredient through the mucosa of the mouth.

(3) The addition of a flavour also allows the person administering the drug to check firstly that the drug has been dispensed (the patient can taste or smell it) and secondly that it has been dispensed into the right place - if the drug were e.g. accidentally dispensed directly into the throat, there would be no taste sensation.

(4) A surprising feature is that the essential oil (especially levomenthol) also assists with the solubilisation of the artemether. In a solubility trial, dissolution of artemether in miglyol occurred after 4 minutes 30 seconds when menthol added before artemether compared to 5 minutes 55 seconds when artemether added before menthol.

As an example, preferred formulations (for sublingual or buccal paediatric use) are given in Tables 3 and 4. For adult use, or for the treatment of some indications, concentrations higher or lower than those exemplified are envisaged. Two different dose concentrations are given suitable for use in a spray delivery system. A number of sprays (i.e. individual spray actuations of 100microlitres) may be given, dependent on the weight of the child to be treated:

*Table 3: 3mg Artemether per actuation*

| Raw Material Item | Weight (g) | % w/w |
|---|---|---|
| Artemether IP | 0.090 | 3.2 |
| Levomenthol *Ph. Eur.* | 0.020 | 0.7 |
| Miglyol® 810 | 2.690 | 96.1 |

*Table 4: 6mg Artemether per actuation*

| Raw Material Item | Weight (g) | % w/w |
|---|---|---|
| Artemether IP | 0.180 | 6.4 |
| Levomenthol *Ph. Eur.* | 0.020 | 0.7 |
| Miglyol® 810 | 2.600 | 92.9 |

Table 5 outlines an example of a preferred dosage regime for paediatric use. Alternative regimes are envisaged, e.g. dosing at 3mg/kg body weight.

*Table 5: Paediatric Dosage Regime*

| Weight of child (kg) | Number of doses at 3mg Dose per spray actuation | Total delivered dose mg/kg | Number of doses at 6mg Dose per spray actuation | Total delivered dose mg/kg |
|---|---|---|---|---|
| 3 | 1 | 1.00 | | |
| 4 | 1 | 0.75 | | |
| 5 | 2 | 1.20 | | |
| 6 | 2 | 1.00 | | |
| 7 | 2 | 0.86 | | |
| 8 | 3 | 1.13 | | |
| 9 | 3 | 1.00 | | |
| 10 | 3 | 0.90 | | |
| 11 | 4 | 1.09 | | |

(continued)

| Weight of child (kg) | Number of doses at 3mg Dose per spray actuation | Total delivered dose mg/kg | Number of doses at 6mg Dose per spray actuation | Total delivered dose mg/kg |
|---|---|---|---|---|
| 12 | 4 | 1.00 | 2 | 1.00 |
| 13 | 4 | 0.92 | 2 | 0.92 |
| 14 | 5 | 1.07 | 2 | 0.86 |
| 15 | 5 | 1.00 | 3 | 1.20 |
| 16 | 5 | 0.94 | 3 | 1.13 |
| 17 | | | 3 | 1.06 |
| 18 | | | 3 | 1.00 |
| 19 | | | 3 | 0.95 |
| 20 | | | 3 | 0.90 |
| 21 | | | 3 | 0.86 |
| 22 | | | 4 | 1.09 |
| 23 | | | 4 | 1.04 |
| 24 | | | 4 | 1.00 |
| 25 | | | 4 | 0.96 |
| 26 | | | 4 | 0.92 |
| 27 | | | 4 | 0.89 |
| 28 | | | 5 | 1.07 |
| 29 | | | 5 | 1.03 |
| 30 | | | 5 | 1.00 |

Formulations for adult use may be prepared at higher concentrations of artemether, such as 150-200 mg/ml. For adult use, individual spray volumes may be larger than the 100microlitre example described here for paediatric use.

Bioavailability of Artemether

The applicant has carried out confidential trials to asses the uptake of the artemether-containing compositions of the present invention when delivered by the sublingual route, by comparison to oral administration by tablet.
Trials were carried out on healthy male adult human volunteers (16 subjects per cohort), and subject to normal ethical approval. Three single-dose regimes according to the present invention were studied, and compared to a regime using oral-dosed tablets, as follows:

Sub-Lingual Spray Regimes

Spray formulations of artemether were prepared as detailed above, and administered, on a single occasion, to a group of volunteers by the sublingual route. A number of successive actuations of the spray were administered, as shown in Table 6, below.

*Table 6 - Dosage Regime for Single Dose Study Sublingual Spray Formulation*

| Test | Formulation | Dose per Actuation (mg) | Number of Actuations | Total Doge (mg) |
|---|---|---|---|---|
| T1 | As Table 3 | 3 | 5 | 15 |
| T2 | As Table 3 | 3 | 10 | 30 |
| T3 | As Table 4 | 6 | 5 | 30 |

Reference Oral Dose

As a reference, a fourth group of volunteers were administered tablets containing artemether, on a single occasion, as shown in Table 7, below.

*Table 7-Dosage Regime for Single Dose Study Oral Tablet Formulation*

| Test | Formulation | Dose per Tablet (mg) | Number of Tablets | Total Doge (mg) |
|------|-------------|----------------------|-------------------|------------------|
| T4 | Tablet | 10 | 3 | 30 |

Following administration of each dosage regime, blood samples were taken from the subjects, and plasma concentrations of artemether and its immediate metabolite dihydroartemesinin were determined, in order to compare bioavailability by the two routes.

Figures 1-6 show mean plasma concentration of artemether following two comparison dose regimes. Figures 7-12 show the corresponding mean plasma concentration of dihydroartemesinin.

Figures 1 and 7 compare regimes T1 (open squares) and T4 (closed circles): 15mg artemether via 5 sublingual spray doses vs. 30mg artemether via tablet.

Figures 2 and 8 compare regimes T2 (open squares) and T4 (closed circles): 30mg artemether via 10 sublingual spray doses vs. 30mg artemether via tablet.

Figures 3 and 9 compare regimes T3 (open squares) and T4 (closed circles): 30mg artemether via 5 sublingual spray doses vs. 30mg artemether via tablet.

Figures 4 and 10 compare regimes T1 (open squares) and T2 (closed circles): 15mg artemether via 5 sublingual spray doses vs. 30mg artemether via 10 sublingual spray doses.

Figures 5 and 11 compare regimes T2 (open squares) and T3 (closed circles): 30mg artemether via 10 sublingual spray doses vs. 30mg artemether via 5 sublingual spray doses.

Figures 6 and 12 compare regimes T1 (open squares) and T3 (closed circles): 15mg artemether via 5 sublingual spray doses vs. 30mg artemether via 5 sublingual spray doses).

Pharmacokinetic data for each of the four dosage regimes are given in Tables 8-11, below:

*Table 8:*

| Test Group T1 Single sublingual administration of 15mg Artemether sublingual spray: 3mg per actuation | | |
|---|---|---|
| | Plasma Artemether | Plasma Dihydroartemesinin |
| Pharmacokinetic Parameters* | (n=16) (mean $\pm$SD) | (n=16) (mean $\pm$SD) |
| $AUC_{0-12}$ (ng.h/mL) | 25.85 $\pm$ 13.88 | 29.63 $\pm$ 11.58 |
| $C_{max}$ (ng/mL) | 16.11 $\pm$ 8.69 | 18.29 $\pm$ 7.52 |
| $T_{max}$ (h) | 1.70 $\pm$ 0.68 | 1.83 $\pm$ 0.68 |
| $t_{1/2}$ (h) | 0.72 $\pm$ 0.30 | |
| $\lambda_z$ (h$^{-1}$) | 1.11 $\pm$ 0.40 | |
| CL/F (ng/h) | 0.74 $\pm$ 0.46 | 0.54 $\pm$ 0.15 |
| V/F (L) | 0.68 $\pm$ 0.33 | 0.51 $\pm$ 0.16 |
| *Key: $AUC_{0-12}$ (ng.h/mL) Area under the concentration curve between 0-12 h. $C_{max}$ (ng/mL) Maximum observed plasma concentration $T_{max}$ (h) Time of observed maximum plasma concentration $t_{1/2}$ (h) Elimination half-life $\lambda_z$ (h$^{-1}$) Elimination rate constant CL/F (ng/h) Apparent clearance rate V/F (L) Apparent volume of distribution | | |

*Table 9: Test Group T2 Single sublingual administration of 30mg Artemether sublingual spray: 3mg per actuation*

| Pharmacokinetic Parameters | Plasma Artemether (n=16) (mean ±SD) | Plasma Dihydroartemesinin (n=16) (mean ±SD) |
|---|---|---|
| $AUC_{0-12}$(ng.h/mL) | 76.60 ± 43.12 | 99.51 ± 50.33 |
| $C_{max}$ (ng/mL) | 32.12 ± 16.39 | 44.11 ± 28.48 |
| $T_{max}$ (h) | 1.73±0.82 | 2.10 ± 1.17 |
| $t_{1/2}$ (h) | 1.39 ± 0.49 | |
| $\lambda_z$ ($h^{-1}$) | 0.56 ± 0.20 | |
| CL/F(ng/h) | 0.56 ± 0.37 | 0.36 ± 0.13 |
| V/F (L) | 1.00 ± 0.55 | 0.72 ± 0.36 |
| Key as Table 8 | | |

*Table 10: Test Group T3 Single sublingual administration of 30mg Artemether sublingual spray: 6mg per actuation*

| Pharmacokinetic Parameters | Plasma Artemether (n=16) (mean ±SD) | Plasma Dihydroartemesinin (n=16) (mean ±SD) |
|---|---|---|
| $AUC_{0-12}$ (ng.h/mL) | 71.11 ± 41.08 | 86.19 ± 27.68 |
| $C_{max}$ (ng/mL) | 35.24 ± 23.91 | 41.14 ± 16.45 |
| $T_{max}$ (h) | 1.67 ± 0.77 | 1.88 ± 0.74 |
| $t_{1/2}$ (h) | 1.40 ± 0.59 | |
| $\lambda_z$ ($h^{-1}$) | 0.59 ± 0.25 | |
| CL/F (ng/h) | 0.63 ± 0.49 | 0.39 ± 0.15 |
| V/F(L) | 1.01 ± 0.49 | 0.91 ± 0.67 |
| Key as Table 8 | | |

*Table 11: Test Group T4 Single oral administration of 30mg Artemether Tablets 10mg per Tablet*

| Pharmacokinetic Parameters | Plasma Artemether (n=16) (mean ±SD) | Plasma Dihydroartemesinin (n=16) (mean ±SD) |
|---|---|---|
| $AUC_{0-12}$ (ng.h/mL) | 34.59 ± 21.01 | 38.49 ± 12.38 |
| $C_{max}$ (ng/mL) | 10.12 ± 7.19 | 10.99 ± 4.39 |
| $T_{max}$ (h) | 1.02 ± 0.86 | 1.39 ± 0.88 |
| $t_{1/2}$ (h) | 3.44 ± 4.26 | |
| $\lambda_z$ ($h^{-1}$) | 0.31 ± 0.15 | |
| CL/F (ng/h) | 1.11 ± 1.01 | 0.76 ± 0.23 |
| V/F (L) | 3.90 ± 2.90 | 2.36 ± 1.26 |
| Key as table 8 | | |

From these preliminary results, it can be seen that comparison of the area under the plasma concentration curve during the 12 hours following the doses ($AUC_{0-12}$), a well-accepted measure of absorption, shows significant and surprisingly higher absorption of artemether when administered sublingually as a spray formulation as disclosed herein by comparison to oral tablet dosing.

For comparison of bioavailability of artemether via the sublingual spray route described herein with administration by oral tablets, we have calculated the F-values, commonly used to compare two dose regimes, generally A and B, for the artemether data, as follows:

$$F_{A-B} = \frac{AUC_A}{AUC_B} \frac{dose_B}{dose_A}$$

The results are as follows:

$$F_{T1-T4} = 1.67 \pm 0.60 \text{ (S.D.)}$$

$$F_{T2-T4} = 2.24 \pm 0.92 \text{ (S.D.)}$$

$$F_{T3-T4} = 2.09 \pm 0.69 \text{ (S.D.)}$$

This indicates that approximately between 1.7 and 2.2 times more artemether was absorbed when administered as a sublingual spray as described herein by comparison to oral administration by tablet, despite the oral dose being twice as large in the first instance. The indicative bioavailability by the sublingual route is therefore at least twice that by the oral route for equivalent doses.

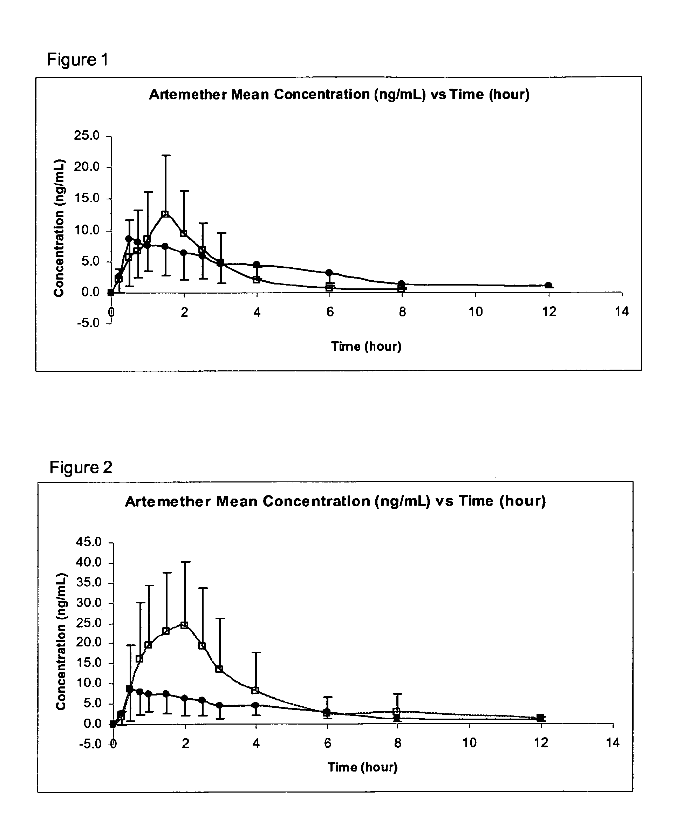

Inspection of the data of Tables 8-11, and Figures 1-12 also confirms this general finding for the primary active metabolite of artemether (dihydroartemesinin).

Avoidance of Autoinduction

It is known that both oral and rectal administration of artemesinins is associated with autoinduction of the drug metabolism in individuals (see e.g. Ashton M, Hai TN, Sy ND, Huong DX, Van Huong N, Nieu NT, Cong LD. "Artemisinin pharmacokinetics is time-dependent during repeated oral administration in healthy male adults. ", Drug Metab Dispos. 1998; 26:25-7, and *"*Retrospective analysis of artemisinin pharmacokinetics: application of a semiphysiological autoinduction model ", Asimus and Gordi, Br. J Clin Pharmacol. 2007 June; 63(6): 758-762). As a result, systemically circulating artemesinin declines with each successive dose, thereby reducing the effectiveness of drug dosage regimes.

In confidential trials, the inventors have found that administration of artemesinins by the transmucosal sublingual route avoids such autoinduction, leading to consistent uptake and accumulating systemic concentration of the active drug metabolite, dihydroartemesinin, thereby providing significant advantage in administration by the sublingual route. A similar avoidance of autoinduction is expected with delivery by the transmucosal buccal or nasal route.

In confidential trials, volunteers followed the following treatment: A single administration of 30mg artemether sublingual spray 6mg/actuation on days 1 and 5 following an overnight fast, and twice daily administrations of 30mg artemether sublingual spray 3mg/actuation on days 2, 3, and 4 following a morning or evening meal. Blood samples were collected for pharmacokinetic analysis at the following time points:

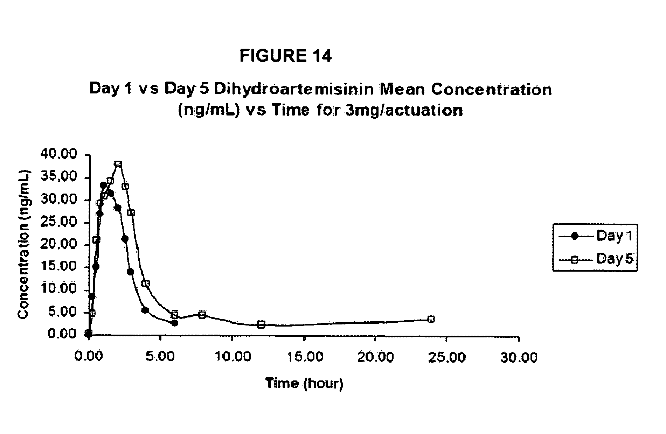

Day 1: Predose, 0.25, 0.5, 0.75, 1, 1.5, 2, 2.5, 3, 4, 6, 8, and 12 h after dosing.
Days 2, 3, and 4: pre morning dose and 0.5, 1, 2 and 4 h after morning dose and pre evening dose and 1 hour after evening dose.
Day 5: Predose, 0.25, 0.5, 0.75, 1, 1.5, 2, 2.5, 3, 4, 6, 8, 12 h and 24 h after dosing. Pharmacokinetic analysis of plasma dihydroartemesinin on days 1 and 5 revealed an effectively identical response, indicating the lack of autoinduction. Plasma concentration curves are shown in Figure 14.

Figures Captions

Figure 1: Plot of mean plasma Artemether concentration vs time with standard deviation following a single sublingual administration of 15mg Artemether Sublingual Spray 3mg/actuation (T1) and single oral administration of 30mg Artemether Tablets 10 mg/tablet (T4). Mean ± SD (● = reference, T4, □ = test, T1)

Figure 2: Plot of mean plasma Artemether concentration vs time with standard deviation following a single sublingual administration of 30mg Artemether Sublingual Spray 3mg/actuation (T2) and single oral administration of 30mg Artemether Tablets 10 mg/tablet (T4). Mean ± SD (● = reference, T4, □ = test, T2)

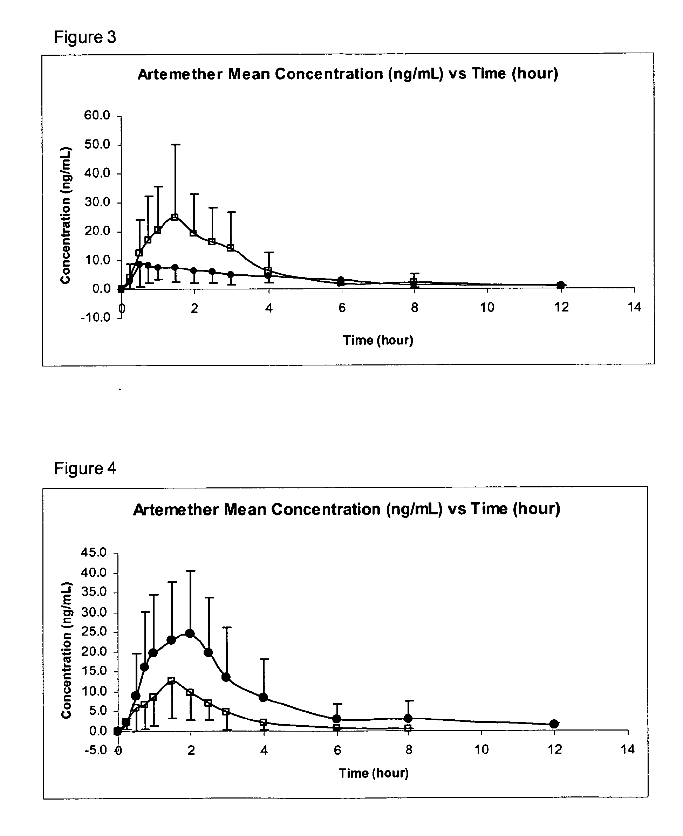

Figure 3: Plot of mean plasma Artemether concentration vs time with standard deviation following a single sublingual administration of 30mg Artemether Sublingual Spray 6mg/actuation (T3) versus single oral administration of 30mg Artemether Tablets 10 mg/tablet (T4). Mean ± SD (● = reference, T4, □ = test, T3)

Figure 4: Plot of mean plasma artemether concentration vs time with standard deviation following a single sublingual administration of 15mg Artemether Sublingual Spray 3mg/actuation (T1) versus single sublingual administration of 30mg Artemether Sublingual Spray 3mg/actuation (T2). Mean ± SD (● = reference, T2 , □ = test, T1)

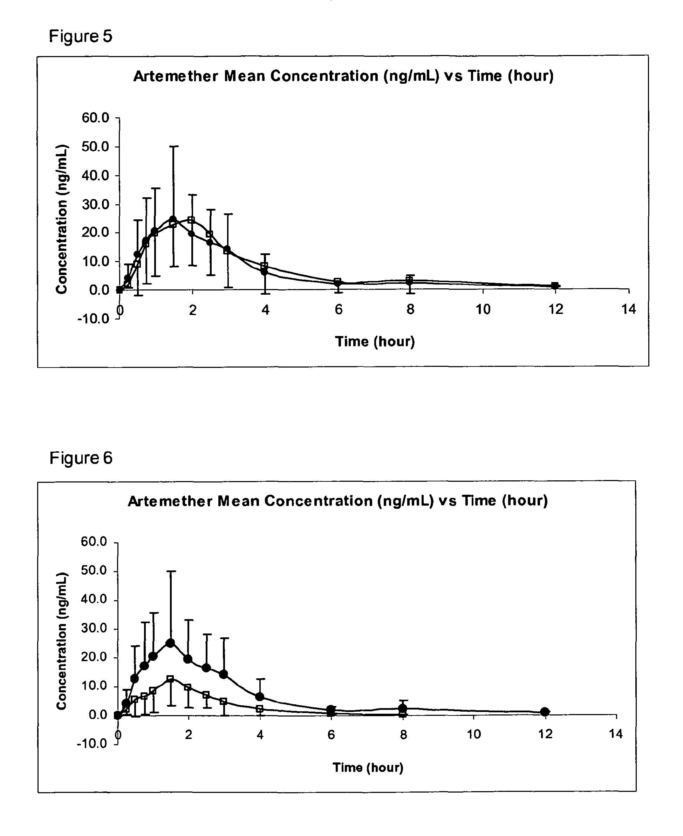

Figure 5: Plot of mean plasma Artemether concentration vs time with standard deviation following a single sublingual administration of 30mg Artemether Sublingual Spray 3mg/actuation (T2) versus single sublingual administration of 30mg Artemether Sublingual Spray 6mg/actuation (T3). Mean ± SD (● = reference, T3 , □ = test, T2)

Figure 6: Plot of mean plasma Artemether concentration vs time with standard deviation following a single sublingual administration of 15mg Artemether Sublingual Spray 3mg/actuation (T1) versus single sublingual administration of 30mg Artemether Sublingual Spray 6mg/actuation (T3). Mean ± SD (● = reference, T3 , □ = test, T1)

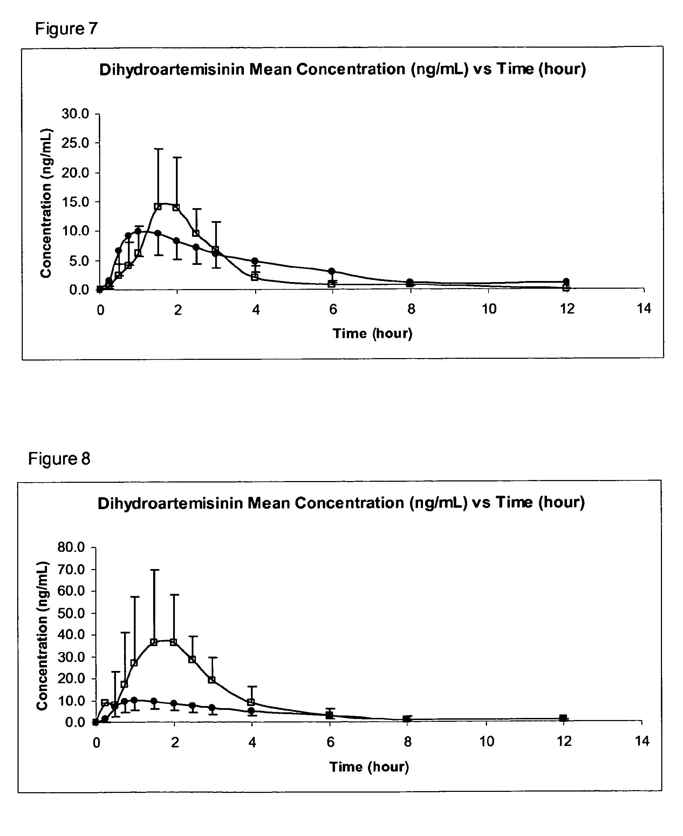

Figure 7: Plot of mean plasma Dihydroartemisinin concentration vs time with standard deviation following a single sublingual administration of 15mg Artemether Sublingual Spray 3mg/actuation (T1) and single oral administration of 30mg Artemether Tablets 10 mg/tablet (T4). Mean ± SD (● = reference, T4, □ = test, T1)

Figure 8: Plot of mean plasma Dihydroartemisinin concentration vs time with standard deviation following a single sublingual administration of 30mg Artemether Sublingual Spray 3mg/actuation (T2) and single oral administration of 30mg Artemether Tablets 10 mg/tablet (T4). Mean ± SD (● = reference, T4, □ = test, T2)

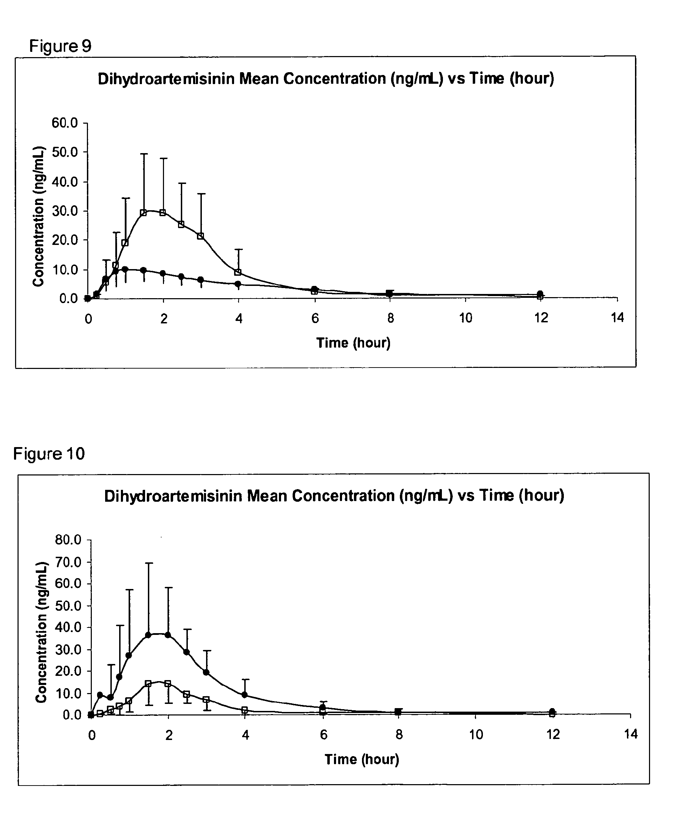

Figure 9: Plot of mean plasma Dihydroartemisinin concentration vs time with standard deviation following a single' sublingual administration of 30mg Artemether Sublingual Spray 6mg/actuation (T3) versus single oral administration of 30mg Artemether Tablets 10 mg/tablet (T4). Mean ± SD (● = reference, T4 , □ = test, T3)

Figure 10: Plot of mean plasma Dihydroartemisinin concentration vs time with standard deviation following a single sublingual administration of 15mg Artemether Sublingual Spray 3mg/actuation (T1) versus single sublingual administration of 30mg Artemether Sublingual Spray 3mg/actuation (T2). Mean ± SD (● = reference, T2 , □ = test, T1)

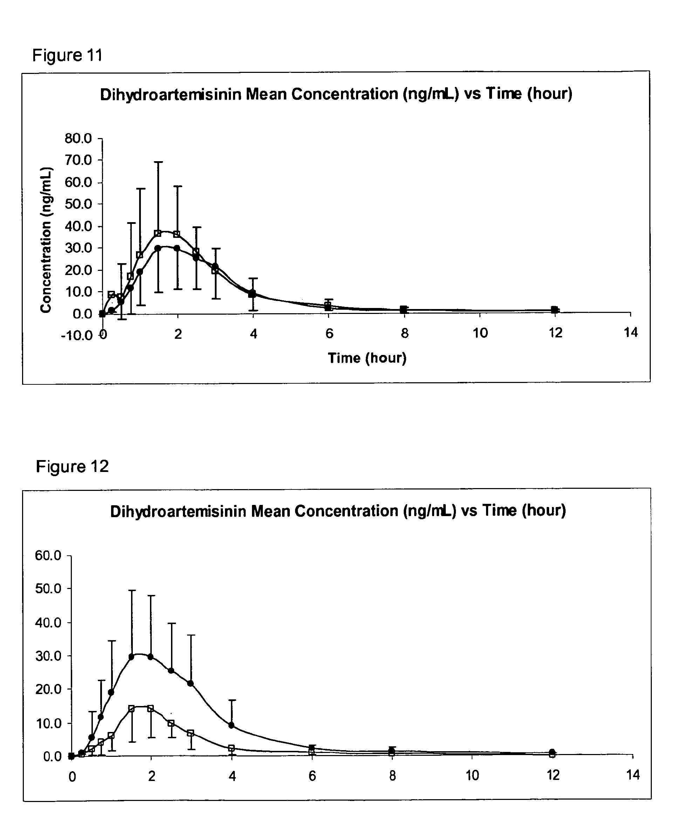

Figure 11: Plot of mean plasma Dihydroartemisinin concentration vs time with standard deviation following a single sublingual administration of 30mg Artemether Sublingual Spray 3mg/actuation (T2) versus single sublingual administration of 30mg Artemether Sublingual Spray 6mg/actuation (T3). Mean ± SD (● = reference, T3 , □ = test, T2)

Figure 12: Plot of mean plasma Dihydroartemisinin concentration vs time with standard deviation following a single sublingual administration of 15mg Artemether Sublingual Spray 3mg/actuation (T1) versus single sublingual administration of 30mg Artemether Sublingual Spray 6mg/actuation (T3). Mean ± SD (● = reference, T3, □ = test, T1)

**Claims**

1. A pharmaceutical composition for use in the treatment of a neoplastic disease, said composition comprising:

   artemesinin, artemether, arteether, artenimol or artesunate; and
   a pharmaceutically-acceptable excipient selected from the group consisting of:

      medium chain length triglycerides;
      short chain triglycerides;

omega-3-marine triglycerides; and
fish oil, rich in omega-3-acids

said composition formulated for transmucosal sublingual, buccal or nasal dosage.

2. A pharmaceutical composition for use according to claim 1 wherein said disease comprises a malignant neoplasm.

3. A pharmaceutical composition for use according to claim 2 wherein said disease is selected from the group consisting of:

pituitary adenoma;
squamous cell carcionoma;
breast cancer;
non-Hodgkin's Lymphoma;
skin cancer;
lung cancer; and
non-small cell lung carcinoma.

4. A pharmaceutical composition for use in the treatment of fluke infestation, said composition comprising:

artemesinin, artemether, arteether, artenimol or artesunate; and
a pharmaceutically-acceptable excipient selected from the group consisting of:

medium chain length triglycerides;
short chain triglycerides;
omega-3-marine triglycerides; and
fish oil, rich in omega-3-acids

said composition formulated for transmucosal sublingual, buccal or nasal dosage.

5. A pharmaceutical composition for use in the treatment of Lyme disease (Borreliosis), said composition comprising:

artemesinin, artemether, arteether, artenimol or artesunate; and
a pharmaceutically-acceptable excipient selected from the group consisting of:

medium chain length triglycerides;
short chain triglycerides;
omega-3-marine triglycerides; and
fish oil, rich in omega-3-acids

said composition formulated for transmucosal sublingual, buccal or nasal dosage.

6. A pharmaceutical composition for use according to any one of claims 1 to 5 wherein said composition consists essentially of:

artemesinin, artemether, arteether, artenimol or artesunate; and
a pharmaceutically-acceptable excipient selected from the group consisting of:

medium chain length triglycerides;
short chain triglycerides;
omega-3-marine triglycerides; and
fish oil, rich in omega-3-acids

said composition formulated for transmucosal sublingual, buccal or nasal dosage.

7. A pharmaceutical composition for use according to any one of claims 1 to 5 wherein said composition consists essentially of:

artemesinin, artemether, arteether, artenimol or artesunate; and
a pharmaceutically acceptable excipient consisting essentially of:
a triglyceride, liquid at 37°C; and
medium chain length triglycerides;

said composition formulated for transmucosal sublingual, buccal or nasal dosage.

8. A pharmaceutical composition for use according to any preceding claim wherein said composition is substantially free of water.

9. A pharmaceutical composition for use according to any preceding claim wherein said composition is substantially free of ethanol.

10. A pharmaceutical composition for use according to any preceding claim wherein said composition further comprises an essential oil.

11. A pharmaceutical composition for use according to any preceding claim wherein said composition is formulated for sublingual delivery.

12. A pharmaceutical composition for use according to any preceding claim wherein said composition is contained within a medicament delivery device, said device adapted to deliver individual or successive doses of said composition, each individual or successive dose having a volume of less than 1000 microlitres.

13. A pharmaceutical composition for use according to claim 12 wherein said device comprises a pump spray.

14. A pharmaceutical composition for use according to any one of claims 1 to 11 wherein said composition is contained within a container comprised within a device for providing pharmaceutical doses with valve means arranged to transfer doses of said pharmaceutical composition to the exterior of the container.

15. A pharmaceutical composition for use according to any one of claims 1 to 11 wherein said composition is contained within a kit that comprises instructions to administer said composition to a patient in need thereof by the transmucosal sublingual, buccal or nasal route.


**Patentansprüche**

1. Pharmazeutische Zusammensetzung zur Verwendung bei der Behandlung einer neoplastischen Erkrankung, die Zusammensetzung umfassend:

Artemesinin, Artemether, Arteether, Artenimol oder Artesunat; und
einen pharmazeutisch akzeptablen Trägerstoff, der aus der Gruppe ausgewählt ist, welche umfasst:

mittelkettige Triglyceride;
kurzkettige Triglyceride;
marine Omega-3-Triglyceride; und
Fischöl, das reich an Omega-3-Säuren ist

wobei die Zusammensetzung zur sublingualen, bukkalen oder nasalen Transmukosal-Dosierung formuliert ist.

2. Pharmazeutische Zusammensetzung zur Verwendung gemäß Anspruch 1, wobei die Erkrankung ein malignes Neoplasma umfasst.

3. Pharmazeutische Zusammensetzung zur Verwendung gemäß Anspruch 2, wobei die Erkrankung aus der Gruppe ausgewählt ist, welche umfasst:

Hypophysenadenom;
Plattenepithelkarzinom;
Brustkrebs;

Non-Hodgkin-Lymphom;
Hautkrebs;
Lungenkrebs; und
nicht-kleinzelliges Lungenkarzinom.

4. Pharmazeutische Zusammensetzung zur Verwendung bei der Behandlung von Plattwurmbefall, die Zusammensetzung umfassend:

Artemesinin, Artemether, Arteether, Artenimol oder Artesunat; und
einen pharmazeutisch akzeptablen Trägerstoff, der aus der Gruppe ausgewählt ist, welche umfasst:

mittelkettige Triglyceride;
kurzkettige Triglyceride;
marine Omega-3-Triglyceride; und
Fischöl, das reich an Omega-3-Säuren ist

wobei die Zusammensetzung zur sublingualen, bukkalen oder nasalen Transmukosal-Dosierung formuliert ist.

5. Pharmazeutische Zusammensetzung zur Verwendung bei der Behandlung der Lyme-Krankheit (Borreliose), die Zusammensetzung umfassend:

Artemesinin, Artemether, Arteether, Artenimol oder Artesunat; und
einen pharmazeutisch akzeptablen Trägerstoff, der aus der Gruppe ausgewählt ist, welche umfasst:

mittelkettige Triglyceride;
kurzkettige Triglyceride;
marine Omega-3-Triglyceride; und
Fischöl, das reich an Omega-3-Säuren ist

wobei die Zusammensetzung zur sublingualen, bukkalen oder nasalen Transmukosal-Dosierung formuliert ist.

6. Pharmazeutische Zusammensetzung zur Verwendung gemäß einem der Ansprüche 1 bis 5, wobei die Zusammensetzung im Wesentlichen besteht aus:

Artemesinin, Artemether, Arteether, Artenimol oder Artesunat; und
einen pharmazeutisch akzeptablen Trägerstoff, der aus der Gruppe ausgewählt ist, welche umfasst:

mittelkettige Triglyceride;
kurzkettige Triglyceride;
marine Omega-3-Triglyceride; und
Fischöl, das reich an Omega-3-Säuren ist

wobei die Zusammensetzung zur sublingualen, bukkalen oder nasalen Transmukosal-Dosierung formuliert ist.

7. Pharmazeutische Zusammensetzung zur Verwendung gemäß einem der Ansprüche 1 bis 5, wobei die Zusammensetzung im Wesentlichen besteht aus:

Artemesinin, Artemether, Arteether, Artenimol oder Artesunat; und
einem pharmazeutisch akzeptablen Trägerstoff, der im Wesentlichen besteht aus:

einem Triglycerid, das bei 37 °C flüssig ist; und
mittelkettigen Triglyceriden;

wobei die Zusammensetzung zur sublingualen, bukkalen oder nasalen Transmukosal-Dosierung formuliert ist.

8. Pharmazeutische Zusammensetzung zur Verwendung gemäß einem der vorherigen Ansprüche, wobei die Zusammensetzung im Wesentlichen frei von Wasser ist.

9. Pharmazeutische Zusammensetzung zur Verwendung gemäß einem der vorherigen Ansprüche, wobei die Zusammensetzung im Wesentlichen frei von Ethanol ist.

10. Pharmazeutische Zusammensetzung zur Verwendung gemäß einem der vorherigen Ansprüche, wobei die Zusammensetzung des Weiteren ein ätherisches Öl umfasst.

11. Pharmazeutische Zusammensetzung zur Verwendung gemäß einem der vorherigen Ansprüche, wobei die Zusammensetzung zur sublingualen Verabreichung formuliert ist.

12. Pharmazeutische Zusammensetzung zur Verwendung gemäß einem der vorherigen Ansprüche, wobei die Zusammensetzung in einer Vorrichtung zur Medikamentenverabreichung enthalten ist, wobei die Vorrichtung so ausgelegt ist, dass sie einzelne oder sukzessive Dosen der Zusammensetzung verabreicht, wobei jede einzelne oder sukzessive Dosis ein Volumen von weniger als 1000 Mikrolitern hat.

13. Pharmazeutische Zusammensetzung zur Verwendung gemäß Anspruch 12, wobei die Vorrichtung ein Pumpspray umfasst.

14. Pharmazeutische Zusammensetzung zur Verwendung gemäß einem der Ansprüche 1 bis 11, wobei die Zusammensetzung in einem Behälter untergebracht ist, der sich in einer Vorrichtung zur Bereitstellung pharmazeutischer Dosen befindet, die über eine Ventileinrichtung verfügt, welche zur Abgabe von Dosen der pharmazeutischen Zusammensetzung aus dem Behälter heraus eingerichtet ist.

15. Pharmazeutische Zusammensetzung zur Verwendung gemäß einem der Ansprüche 1 bis 11, wobei die Zusammensetzung in einem Kit untergebracht ist, das Anleitungen zur Verabreichung der Zusammensetzung an einen behandlungsbedürftigen Patienten über den sublingualen, bukkalen oder nasalen Transmukosal-Weg umfasst.


**Revendications**

1. Une composition pharmaceutique pour une utilisation dans le traitement d'une maladie néoplasique, ladite composition comportant :

artémisinine, artéméther, artééther, arténimol ou artésunate ; et
un excipient de qualité pharmaceutique sélectionné dans le groupe composé de :

triglycérides de longueur de chaîne moyenne ;
triglycérides à chaîne courte ;
triglycérides marins oméga-3 ; et
huile de poisson, riche en acides oméga-3

ladite composition formulée pour un dosage sublingual transmuqueux, buccal ou nasal.

2. Une composition pharmaceutique pour une utilisation selon la revendication 1 dans laquelle ladite maladie comporte une tumeur maligne.

3. Une composition pharmaceutique pour une utilisation selon la revendication 2 dans laquelle ladite maladie est sélectionnée dans le groupe composé de :

adénome pituitaire ;
carcinome malpighien ;
cancer du sein ;
lymphome non hodgkinien ;
cancer de la peau ;
cancer du poumon ; et
carcinome pulmonaire non à petites cellules.

4. Une composition pharmaceutique pour une utilisation dans le traitement de la distomatose, ladite composition comportant :

artémisinine, artéméther, artééther, arténimol ou artésunate ; et
un excipient de qualité pharmaceutique sélectionné dans le groupe composé de:

triglycérides de longueur de chaîne moyenne ;
triglycérides à chaîne courte ;
triglycérides marins oméga-3 ; et
huile de poisson, riche en acides oméga-3

ladite composition formulée pour un dosage sublingual transmuqueux, buccal ou nasal.

5. Une composition pharmaceutique pour une utilisation dans le traitement de la maladie de Lyme (borréliose), ladite composition comportant :

artémisinine, artéméther, artééther, arténimol ou artésunate ; et
un excipient de qualité pharmaceutique sélectionné dans le groupe composé de:

triglycérides de longueur de chaîne moyenne ;
triglycérides à chaîne courte ;
triglycérides marins oméga-3 ; et
huile de poisson, riche en acides oméga-3

ladite composition formulée pour un dosage sublingual transmuqueux, buccal ou nasal.

6. Une composition pharmaceutique pour une utilisation selon n'importe laquelle des revendications 1 à 5 dans laquelle ladite composition se compose essentiellement de :

artémisinine, artéméther, artééther, arténimol ou artésunate ; et
un excipient de qualité pharmaceutique sélectionné dans le groupe composé de:

triglycérides de longueur de chaîne moyenne ;
triglycérides à chaîne courte ;
triglycérides marins oméga-3 ; et
huile de poisson, riche en acides oméga-3

ladite composition formulée pour un dosage sublingual transmuqueux, buccal ou nasal.

7. Une composition pharmaceutique pour une utilisation selon n'importe laquelle des revendications 1 à 5 dans laquelle ladite composition se compose essentiellement de :

artémisinine, artéméther, artééther, arténimol ou artésunate ; et
un excipient de qualité pharmaceutique composé essentiellement de :
un triglycéride, liquide à 37 °C; et
triglycérides de longueur de chaîne moyenne ;

ladite composition formulée pour un dosage sublingual transmuqueux, buccal ou nasal.

8. Une composition pharmaceutique pour une utilisation selon n'importe quelle revendication précédente dans laquelle ladite composition est substantiellement dépourvue d'eau.

9. Une composition pharmaceutique pour une utilisation selon n'importe quelle revendication précédente dans laquelle ladite composition est substantiellement dépourvue d'éthanol. 1.

10. Une composition pharmaceutique pour une utilisation selon n'importe quelle revendication précédente dans laquelle ladite composition comporte en outre une huile essentielle.

11. Une composition pharmaceutique pour une utilisation selon n'importe quelle revendication précédente dans laquelle ladite composition est formulée pour une administration sublinguale.

**12.** Une composition pharmaceutique pour une utilisation selon n'importe quelle revendication précédente dans laquelle ladite composition est contenue à l'intérieur d'un dispositif d'administration de médicament, ledit dispositif étant adapté pour administrer des doses individuelles ou successives de ladite composition, chaque dose individuelle ou successive ayant un volume de moins de 1 000 microlitres.

**13.** Une composition pharmaceutique pour une utilisation selon la revendication 12 dans laquelle ledit dispositif comporte un vaporisateur à pompe.

**14.** Une composition pharmaceutique pour une utilisation selon n'importe laquelle des revendications 1 à 11 dans laquelle ladite composition est contenue à l'intérieur d'un contenant compris à l'intérieur d'un dispositif destiné à fournir des doses pharmaceutiques avec un moyen formant valve agencé pour transférer des doses de ladite composition pharmaceutique à l'extérieur du contenant.

**15.** Une composition pharmaceutique pour une utilisation selon n'importe laquelle des revendications 1 à 11 dans laquelle ladite composition est contenue à l'intérieur d'un kit qui comporte des instructions pour administrer ladite composition à un patient qui en a besoin par voie sublinguale transmuqueuse, buccale ou nasale.

Figure 1

Artemether Mean Concentration (ng/mL) vs Time (hour)

Figure 2

Artemether Mean Concentration (ng/mL) vs Time (hour)

Figure 3

Figure 4

Figure 5

Artemether Mean Concentration (ng/mL) vs Time (hour)

Figure 6

Artemether Mean Concentration (ng/mL) vs Time (hour)

Figure 7

Dihydroartemisinin Mean Concentration (ng/mL) vs Time (hour)

Figure 8

Dihydroartemisinin Mean Concentration (ng/mL) vs Time (hour)

Figure 9

Dihydroartemisinin Mean Concentration (ng/mL) vs Time (hour)

Figure 10

Dihydroartemisinin Mean Concentration (ng/mL) vs Time (hour)

Figure 11

**Dihydroartemisinin Mean Concentration (ng/mL) vs Time (hour)**

Figure 12

**Dihydroartemisinin Mean Concentration (ng/mL) vs Time (hour)**

# Figure 13: Representative chromatograms

a. Aerosol Formulation (20% v/v Ethanol; 80% v/v propellant)

b. Aerosol Formulation (50% v/v Ethanol; 50% v/v propellant)

c. Pump Spray formulation (Ethanol solvent)

c. Pump Spray formulation (Miglyol solvent)

## FIGURE 14

### Day 1 vs Day 5 Dihydroartemisinin Mean Concentration (ng/mL) vs Time for 3mg/actuation

EP 2 424 523 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Sing ; Panwar.** *Integrative Cancer Therapies,* 2006, vol. 5 (4), 391-394
- **Singh ; Verma.** *Archive of Oncology,* 2002, vol. 10 (4), 279-280
- **Singh ; Lai.** *Life Sciences,* 2001, vol. 70, 49-56
- **Efferth et al.** Anti-malaria drug is also active against cancer. *Int. J. Oncology,* 2001, vol. 18, 767-773
- **Keiser ; Morson.** *Exp. Parasitol.,* 2008, vol. 118 (2), 228-37
- **Keiser et al.** *J. Antimicrobial Chemotherapy,* 2006, vol. 57, 1139-1145
- **Utzinger et al.** *Curr Opin Investig Drugs,* 08 February 2007, 105-16
- **Woodrow et al.** *Postgrad. Med.J.,* 2005, vol. 81, 71-78
- **Navaratnam et al.** *Clin Pharmacokinet,* October 2000, vol. 39 (4), 255-270
- **Ashton M ; Hai TN ; Sy ND ; Huong DX ; Van Huong N ; Nieu NT ; Cong LD.** Artemisinin pharmacokinetics is time-dependent during repeated oral administration in healthy male adults. *Drug Metab Dispos,* 1998, vol. 26, 25-7
- **Asimus ; Gordi.** Retrospective analysis of artemisinin pharmacokinetics: application of a semiphysiological autoinduction model. *Br. J Clin Pharmacol.,* June 2007, vol. 63 (6), 758-762